# EUROPÄISCHE PATENTSCHRIFT

(11) **EP 0 304 773 B1**
(45) Veröffentlichungstag und Bekanntmachung des Hinweises auf die Patenterteilung: **17.02.1993**
(21) Anmeldenummer: 88113276.5
(22) Anmeldetag: 16.08.1988
(51) Int. Cl.: A61B 6/06, G21K 1/04

(54) **Primärstrahlenblende für Röntgendiagnostikgeräte**
Diaphragm for primary radiation in an X-ray diagnostic apparatus
Diaphragme pour rayonnement primaire dans un appareil de radiodiagnostic

(30) Priorität: 26.08.1987 DE 8711572 U
(43) Veröffentlichungstag der Anmeldung: 01.03.1989
(73) Patentinhaber: SIEMENS AKTIENGESELLSCHAFT, 80333 München (DE)
(72) Erfinder: Schäfer, Willi, D-8520 Erlangen (DE); Bartmann, Günter, D-8520 Erlangen (DE); Meyer, Michael, D-8581 Eckersdorf (DE)

(56) Entgegenhaltungen:
- EP-A- 0 184 695
- EP-A- 0 188 783
- FR-A- 2 530 857
- US-A- 3 829 701

## Beschreibung

Die Erfindung betrifft eine Primärstrahlenblende gemäß dem ersten Teil des Patentanspruches 1.

Durch die FR-A-2 530 857 ist eine Primärstrahlenblende für ein Röntgendiagnostikgerät bekannt, bei der die Blendenplatten einzeln auf einem mit Längsschlitzen versehenen Tragrahmen einstellbar sind. Sie werden in der jeweils gewählten Stellung durch Schrauben gesichert. Die Einstellung ist deshalb kompliziert und aufwendig. Außerdem ist eine Ferneinstellung mit Hilfe einer gesonderten Bedienvorrichtung nicht möglich.

Der Erfindung liegt die Aufgabe zugrunde, eine Primärstrahlenblende für ein Röntgendiagnostikgerät gemäß dem ersten Teil des Patentanspruches 1 so auszubilden, daß eine besonders einfache Verstellung der Blende anhand der Bedienvorrichtung möglich ist.

Diese Aufgabe ist erfindungsgemäß gelöst durch den zweiten Teil des Patentanspruches 1. Bei der erfindungsgemäßen Primärstrahlenblende betätigt der Arzt die Bedienvorrichtung in dem Sinne, in dem die Blende eingestellt werden soll.

Eine Weiterbildung der Erfindung besteht darin, daß an den die Strahlung begrenzenden Kanten der Blendenplatten halbtransparente Blendenstücke mittels eines Hebelgestänges senkrecht zur jeweiligen Kante derart verstellbar sind, daß der halbtransparente Bereich in Abhängigkeit von der Blendenöffnung eingestellt wird. Bei der bekannten Primärstrahlenblende ist ein halbtransparenter Bereich an den Blendenkanten vorgesehen, der noch von Röntgenstrahlung durchdrungen wird, jedoch die Röntgenstrahlung bereits sichtbar schwächt. Im halbtransparenten Bereich sind dabei Instrumente noch sichtbar, die z.B. in ein Operationsfeld eingeführt werden, so daß das Zielen für den Arzt erleichtert ist. Andererseits werden für die Diagnostik unwichtige Bereiche des Patienten nicht unnötig von Röntgenstrahlung durchdrungen. Bei der erfindungsgemäßen Weiterbildung ist nun der halbtransparente Bereich nicht fest vorgegeben, sondern wird automatisch in Abhängigkeit von der Blendenöffnung eingestellt. Er muß nämlich bei kleinem Schlitz größer sein als bei großem Schlitz, was bei dieser Weiterbildung in einfacher Weise erzielbar ist.

Die Erfindung ist nachfolgend anhand eines in der Zeichnung dargestellten Ausführungsbeispieles näher erläutert. Es zeigen:
- Fig. 1 bis 3: verschiedene Darstellungen einer Primärstrahlenblende nach der Erfindung, und
- Fig. 4: die Primärstrahlenblende gemäß den Fig. 1 bis 3 in Verbindung mit dem zugeordneten Röntgendiagnostikgerät und einer Bedienvorrichtung.

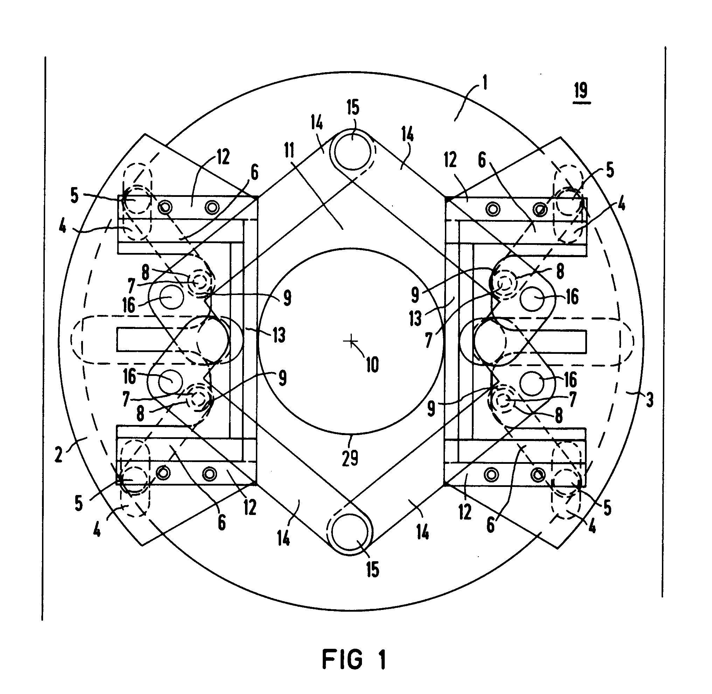

Die Primärstrahlenblende gemäß den Fig. 1 bis 3 weist einen drehbaren Ring 1 auf, auf dem zwei Blendenplatten 2, 3 aufeinander zu und voneinander weg verstellbar gelagert sind. Hierzu sind die Blendenplatten 2, 3 mit Langlöchern 4 versehen, in denen Stifte 5 verschiebbar gelagert sind, welche an Hebeln 6 befestigt sind. Die Hebel 6 sind um Achsen 7 mit Hilfe von Elektromotoren 8 drehbar gelagert. Durch die Elektromotoren 8 können demgemäß die Hebel 6 verschwenkt und damit die Blendenplatten 2, 3 verstellt werden. Da jeder Hebel 6 individuell verschwenkbar ist, ist es möglich, die Blendenplatten 2, 3 nicht nur radial zum Zentralstrahl 10, sondern auch schräg dazu zu verstellen. Ferner können aufgrund der vier Elektromotore 8 die Blendenplatten 2, 3 individuell verstellt werden.

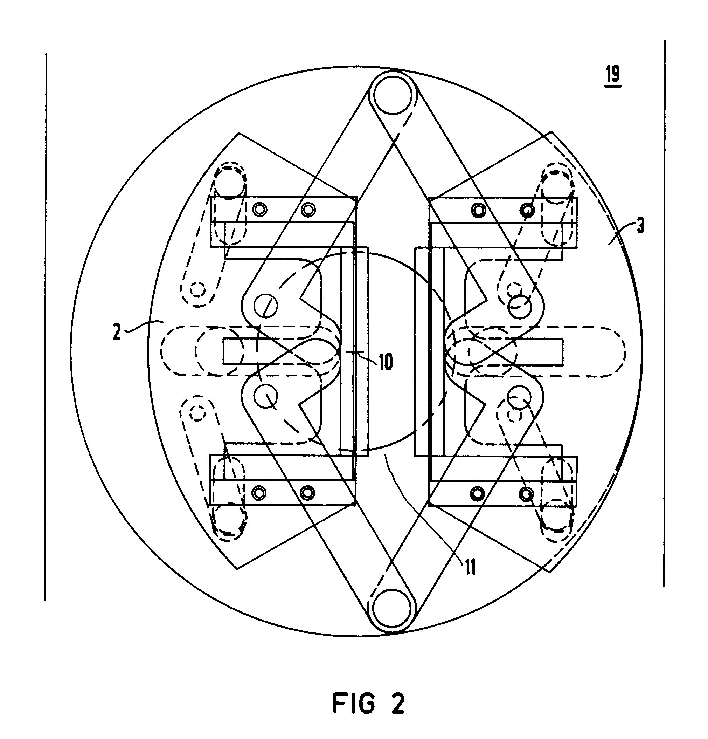

Die Fig. 2 zeigt eine Stellung der Blendenplatten 2, 3, in der der Schlitz 11, der durch die Blendenplatten 2, 3 begrenzt wird, unsymmetrisch zum Zentralstrahl 10 liegt.

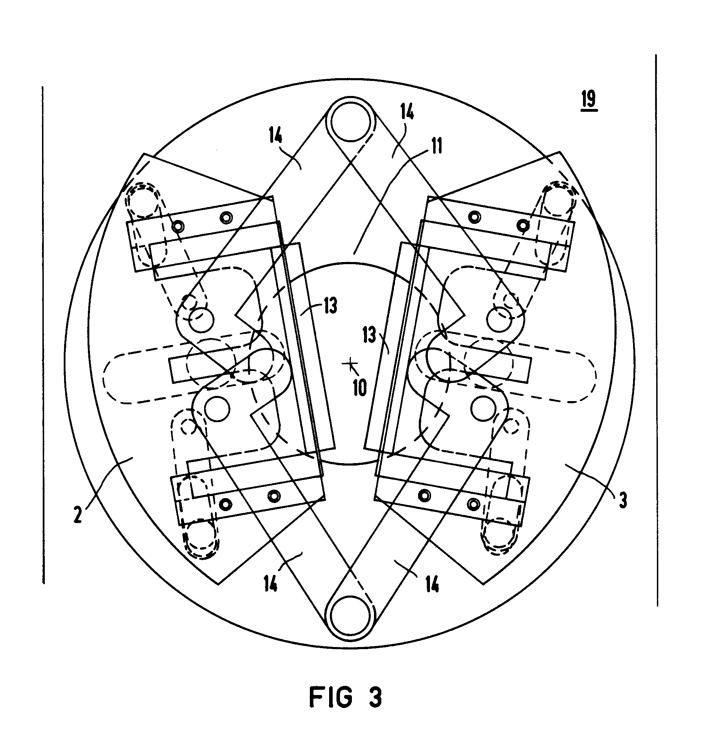

Die Fig. 3 zeigt eine Stellung der Blendenplatten 2, 3, in der diese einen V-förmigen Schlitz 11 begrenzen.

Durch Drehung des Ringes 1 mit den Blendenplatten 2, 3 ist eine weitere Einstellmöglichkeit für den Schlitz 11 gegeben.

Aus den Fig. 1 bis 3 ergibt sich, daß der durch die Blendenplatten 2, 3 begrenzte Schlitz 11 beliebig zum Zentralstrahl 10, also auch unsymmetrisch dazu, liegen und sowohl rechteckig als auch V-förmig sein kann. Dies ist dadurch erreicht, daß die Blendenplatten 2, 3 individuell zweidimensional verstellbar sind.

Aus den Fig. 1 bis 3 geht hervor, daß die Blendenplatten 2, 3 mit Führungen 12 versehen sind, in denen halbtransparente Blendenstücke 13 senkrecht zu den die Strahlung begrenzenden Kanten der Blendenplatten 2, 3 verstellbar gelagert sind. Zur Verstellung der Blendenstücke 13 sind Winkelhebel 14 vorgesehen, die mit Hilfe von Gelenken 15 am Ring 1 und mit Hilfe von Gelenken 16 an den Blendenplatten 1, 2 angelenkt sind. Sie liegen mit ihren freien Enden an der Innenseite der halbtransparenten Blendenstücke 13 an, die durch nicht dargestellte Federmittel gegen diese freien Enden gedrückt werden. Aus der Fig. 2 geht beispielsweise hervor, daß beim Schließen der Blendenplatten 2, 3 die freien Enden der Winkelhebel 14 die Blendenstücke 13 nach innen drücken, so daß der halbtransparente Bereich in Abhängigkeit von der Blendenöffnung derart eingestellt wird, daß er beim Schließen der Blende größer wird.

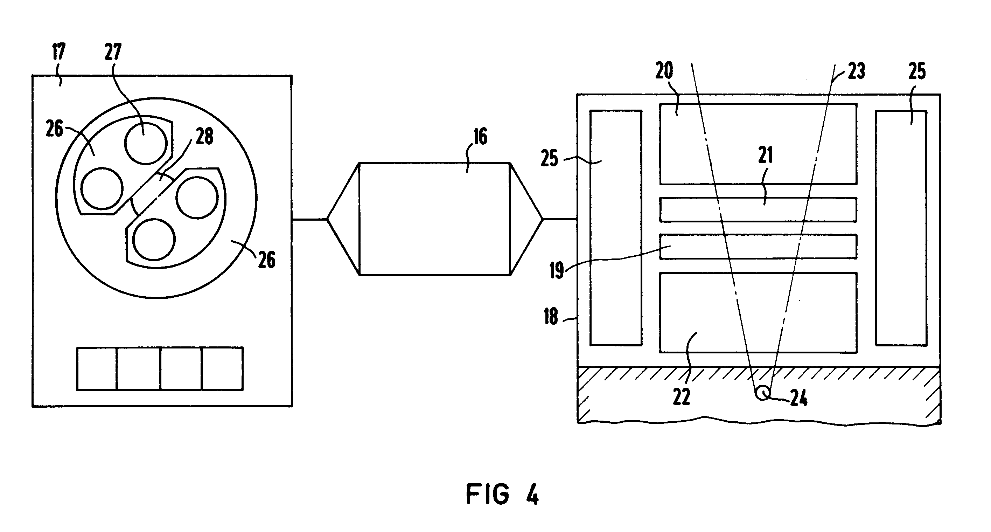

Aus den Fig. 1 bis 3 ist noch erkennbar, daß den Achsen 7 Potentiometer 9 zugeordnet sind, die elektrische Signale liefern, die den Stellungen der Hebel 6 und damit den Stellungen der Blendenplatten 2, 3 entsprechen. Diese elektrische Signale werden in einer in der Fig. 4 dargestellten Nachlaufsteuervorrichtung 16 in dem Sinne verarbeitet, daß die Blende die durch eine Bedienvorrichtung 17 vorgegebene Stellung hat. Die Fig. 4 zeigt schematisch das Gehäuse 18 der Primärstrahlenblende an einem Röntgenuntersuchungsgerät, in dem außer der Primärstrahlenblende 19, die gemäß den Fig. 1 bis 3 aufgebaut ist, noch eine Irisblende 20, eine Festblende 21 für ein vorgegebenes Filmformat und eine Schlitzblende 22 für die Einblendung rechteckiger Blendenöffnungen vorhanden sind. In der Fig. 4 ist die Röntgenstrahlung 23, die vom Fokus 24 einer Röntgenröhre ausgeht, dargestellt. Im Gehäuse 18 sind noch schematisch die Antriebe 25 mit der zugeordneten Elektronik dargestellt.

Aus der Fig. 4 ergibt sich, daß die Bedienvorrichtung 17 zwei Bedienplatten 26 aufweist, die mit Hilfe eines Hebelgestänges, das dem Hebelgestänge der Fig. 1 bis 3 entspricht, in der gleichen Weise verstellbar sind wie die Blendenplatten 2, 3. Die Bedienplatten 26 besitzen Aussparungen 27 für die Finger der Bedienperson, durch die sie leicht verstellbar sind. Ein Kreis 28, der dem Kreis 29 in den Fig. 1 bis 3 entspricht, deutet die maximale Blendenöffnung an. Den Hebeln für die Führung der Bedienplatten 26 sind analog zu den Potentiometern 9 in den Fig. 1 bis 3 Potentiometer zugeordnet, die Sollwertsignale für die Blendenöffnung liefern. Diese Sollwertsignale werden in der Nachlaufsteuervorrichtung 16 mit den von den Potentiometern 9 gelieferten Istwertsignalen verglichen und die Motoren 8 in der Weise angesteuert, daß der Istwert der Blendenöffnung dem eingestellten Sollwert angeglichen wird.

## Patentansprüche

1. Primärstrahlenblende für ein Röntgendiagnostikgerät mit zwei einen Schlitz (11) begrenzenden, in einer Ebene verstellbar gelagerten Blendenplatten (2, 3), welche zweidimensional individuell verstellbar gelagert sind, **dadurch gekennzeichnet,** daß die Blendenplatten (2, 3), die durch eine Bedienvorrichtung (17) gesteuert werden, motorisch verstellbar sind und daß die Bedienvorrichtung (17) zwei Bedienplatten (26) aufweist, die analog zu den Blendenplatten (2, 3) verstellbar gelagert und über eine Nachlaufsteuervorrichtung (16) mit den Motoren (8) für die Verstellung der Blendenplatten (2, 3) elektrisch gekoppelt sind.

2. Primärstrahlenblende nach Anspruch 1, **dadurch gekennzeichnet,** daß an den die Strahlung (23) begrenzenden Kanten der Blendenplatten (2, 3) halbtransparente Blendenstücke (13) mittels eines Hebelgestänges (14) senkrecht zur jeweiligen Kante derart verstellbar angebracht sind, daß der halbtransparente Bereich in Abhängigkeit von der Blendenöffnung eingestellt wird.

3. Primärstrahlenblende nach Anspruch 2, **dadurch gekennzeichnet,** daß das Hebelgestänge (14) so ausgebildet ist, daß der halbtransparente Bereich beim Schließen der Blende (19) größer wird.

## Claims

1. Primary radiation diaphragm for an X-ray diagnostics device having two diaphragm plates (2, 3) defining an aperture (11) and mounted adjustably in a plane, which are individually adjustably mounted in two dimensions, characterised in that the diaphragm plates (2, 3), which are controlled by an operating mechanism (17), are adjustable by motor, and in that the operating mechanism (17) has two operating plates (26) which are adjustably mounted like the diaphragm plates (2, 3) and are electrically coupled by means of a feedback control device (16) to the motors (8) for the adjustment of the diaphragm plates (2, 3).

2. Primary radiation diaphragm according to claim 1, characterised in that semi-transparent diaphragm sections (13) at the edges of the diaphragm plates (2, 3) limiting the radiation (23) are adjustably attached perpendicularly to the respective edge by means of an angle lever (14) so that the semi-transparent region is adjusted dependent on the diaphragm aperture.

3. Primary radiation diaphragm according to claim 2, characterised in that the angle lever (14) is constructed so that the semi-transparent region becomes larger when said diaphragm (19) is closed.

## Revendications

1. Diaphragme du rayonnement primaire pour un appareil de radiodiagnostic, comportant deux plaques de diaphragme (2,3), qui limitent une fente (11), sont montées de manière à être déplaçables dans un plan et à être déplaçables individuellement dans deux dimensions, caractérisé par le fait que les plaques de diaphragme (2,3), qui sont commandées par un dispositif de commande (17), sont réglables au moyen de moteurs et que le dispositif de commande (17) comporte deux plaques de commande (26) qui sont montées de manière à être déplaçables d'une manière analogue aux plaques de diaphragme (2,3) et sont accouplées électriquement, par l'intermédiaire d'un dispositif de commande à asservissement (16), aux moteurs (8) prévus pour déplacer les plaques de diaphragme (2,3).

2. Diaphragme de rayonnement primaire suivant la revendication 1, caractérisé par le fait que des éléments semi-transparents de diaphragme (13) sont montés, au moyen d'une tringlerie à leviers (14), perpendiculairement aux bords respectifs, qui limitent le rayonnement (23), des plaques de diaphragme (2,3), en sorte que la zone semi-transparente est réglée en fonction de l'ouverture du diaphragme.

3. Diaphragme de rayonnement primaire suivant la revendication 2, caractérisé par le fait que la tringlerie à leviers (14) est agencée de telle sorte que la taille de la zone semi-transparente augmente lors de la fermeture du diaphragme (19).
